# EUROPEAN PATENT APPLICATION

(11) **EP 1 908 400 A1**
(43) Date of publication of application: **09.04.2008**
(21) Application number: 06768334.2
(22) Date of filing: 21.07.2006
(51) Int. Cl.: A61B 5/022

(54) **BLOOD PRESSURE VALUE DISPLAY DEVICE, BLOOD PRESSURE MEASURING INSTRUMENT, AND BLOOD PRESSURE VALUE DISPLAY METHOD**

(30) Priority: 21.07.2005 JP 2005211328
(71) Applicant: ICST Corporation, Saitama-shi, Saitama 330-0073 (JP)
(72) Inventor: YOKOI, Hiroyuki, Saitama-shi, Saitama 330-0073 (JP)
(74) Representative: TBK-Patent
(86) International application number: PCT/JP2006/314456
(87) International publication number: WO 2007/011014

(57) **Abstract**

A blood pressure level display device or the like is provided which allows easy measurement and has an improved convenience during measurement.
It has a blood pressure display panel 24 having a circumferentially-calibrated scale, and includes: a systolic blood pressure indicating unit for providing a systolic blood pressure indication based on a state of systolic blood pressure; and a diastolic blood pressure indicating unit for providing a diastolic blood pressure indication based on a state of diastolic blood pressure. It further includes: a high blood pressure side indicator needle 26 which rotates in conjunction with a state of pressure applied to a subject to be measured, and displays at rest a systolic blood pressure level based on the systolic blood pressure indication from the systolic blood pressure indicating unit; and a low blood pressure side indicator needle 28 which displays at rest a diastolic blood pressure level based on the diastolic blood pressure indication from the diastolic blood pressure indicating unit.

## Description

### TECHNICAL FIELD

The present invention relates to a blood pressure level display device and the like to be used in a blood pressure measuring apparatus for measuring blood pressure.

### BACKGROUND ART

Conventionally, there have been developed various types of blood pressure measuring apparatuses, including a mercury column type which uses the climbing level of mercury for measurement, an aneroid type which uses a diaphragm or the like for mechanical measurement, and an electric type which uses various electric control devices for measurement. These blood pressure measuring apparatuses implement a blood pressure level display device so that the blood pressure level can be grasped visually. Among examples of this blood pressure display device are an indicator needle type and a numerical display type. The indicator needle type is one in which an indicator needle is relatively rotated with respect to a blood pressure display panel which has a circumferentially-calibrated scale intended for blood pressure levels, and the blood pressure level is grasped from a point on the scale indicated by this indicator needle. The numerical display type, on the other hand, digitally displays concrete blood pressure levels directly. With the recent development of electronization, the numerical display type for displaying blood pressure levels in concrete numbers has become prevalent. Blood pressure level display devices of numerical display type are highly convenient in being capable of providing clear readings of concrete numerical values instantly, like "a systolic blood pressure of 110 mmHg and a diastolic blood pressure of 70 mmHg."

Blood pressure measuring apparatuses of aneroid type often use a blood pressure level display device of indicator needle type. When measuring blood pressure levels, a cuff having an appropriate width is attached to the periphery of an upper arm to be measured, and air is introduced into the cuff from a pump to increase the cuff pressure (applied pressure) with a stethoscope put on the center of the elbow bending. The blood pressure level display device (meter) is subsequently observed while gradually lowering the cuff pressure. The indicator needle of the blood pressure level display device rotates in the direction of increase of the blood pressure level in conjunction with this cuff pressure, and then slowly rotates in the direction of decrease. The pressure at which a pulse sound is first heard is the systolic blood pressure. The indicator needle is thus checked for a reading on the scale, which is memorized in one's head. Then, the cuff pressure is lowered further until pulse sounds are no longer heard. At that instant the indicator needle is checked for a reading on the scale, which is memorized as a diastolic blood pressure. These systolic blood pressure level and diastolic blood pressure level are written on a memo pad or the like to complete the measurement.

### DISCLOSURE OF THE INVENTION

### PROBLEM TO BE SOLVED BY THE INVENTION

With aneroid blood pressure measuring apparatuses of this type, however, it is necessary to remember the systolic blood pressure and the diastolic blood pressure in one's head while closely observing the indicator needle that moves constantly. This has caused such troubles as forgetting the numerical values halfway, and the operability has not always been high.

In the meantime, blood pressure level display devices of numerical display type require that the blood pressure levels displayed be understood in one's head and compared to typical reference values to determine whether or not the numerical values are favorable. There has thus been the problem of taking long to analyze the blood pressure levels. For example, even in other fields such as clocks for displaying time and car-mount speedometers for indicating speed, display devices of indicator needle type which is visually understandable also continue being used widely. The reason for this is that the display using a needle angle provides the advantage of allowing quicker, intuitive or visual understanding than with concrete numbers. Despite the foregoing problem, the indicator needle type has thus been used for many blood pressure measuring apparatuses.

The present invention has been achieved in view of the foregoing, and it is an object thereof to improve the measuring convenience of a blood pressure level display device of indicator needle type and like significantly, and enhance the original advantage of the indicator needle type.

### MEANS FOR SOLVING THE PROBLEM

The inventor has achieved the foregoing object by the following means.

(1) A blood pressure level display device having a blood pressure display panel provided with a circumferentially-calibrated scale intended for blood pressure levels, with respect to which an indicator needle for displaying a blood pressure level rotates so that the blood pressure level is visually identifiable from the scale indicated by the indicator needle, the device including: a systolic blood pressure indicating unit for providing a systolic blood pressure indication based on a state of systolic blood pressure; a diastolic blood pressure indicating unit for providing a diastolic blood pressure indication based on a state of diastolic blood pressure; a high blood pressure side indicator needle which rotates in conjunction with a state of pressure applied to a subject to be measured, is allowed to stop based on the systolic blood pressure indication from the systolic blood pressure indicating unit, and displays at rest a systolic blood pressure level on the scale; and a low blood pressure side indicator needle which rotates in conjunction with the state of pressure applied to the subject to be measured, is allowed to stop based on the diastolic blood pressure indication from the diastolic blood pressure indicating unit, and displays at rest a diastolic blood pressure level on the scale.

(2) The blood pressure level display device according to the foregoing (1), including: a high blood pressure side numerical area for determining significance of the systolic blood pressure level pointed by the high blood pressure side indicator needle, being circumferentially arranged along the scale; and a low blood pressure side numerical area for determining significance of the diastolic blood pressure level pointed by the low blood pressure side indicator needle, being circumferentially arranged along the scale, and wherein a result of blood pressure measurement can be determined visually from a stop position of the high blood pressure side indicator needle in the high blood pressure side numerical area and a stop position of the low blood pressure side indicator needle in the low blood pressure side numerical area.

(3) The blood pressure level display device according to the foregoing (2), wherein the high blood pressure side numerical area and the low blood pressure side numerical area are each sectioned into at least three types of areas including a high area, a middle area, and a low area.

(4) The blood pressure level display device according to the foregoing (1), (2), or (3), wherein the high blood pressure side indicator needle is a long hand, and the low blood pressure side indicator needle is a short hand.

(5) The blood pressure level display device according to the foregoing (2), wherein the low blood pressure side numerical area is displayed radially inside the high blood pressure side numerical area so that a needlepoint of the high blood pressure side indicator needle, the long hand, falls within the high blood pressure side numerical area and a needlepoint of the low blood pressure side indicator needle, the short hand, falls within the low blood pressure side numerical area.

(6) The blood pressure level display device according to any one of the foregoing (1) to (5), including blood pressure difference display means fixed to either one of the high blood pressure side indicator needle and the low blood pressure side indicator needle, a blood pressure difference between the systolic blood pressure and the diastolic blood pressure being identifiable based on a relative angular difference between the high blood pressure side indicator needle and the low blood pressure side indicator needle at rest.

(7) The blood pressure level display device according to any one of the foregoing (1) to (6), including: a rotating basic shaft for rotating in conjunction with the state of pressure applied to the subject to be measured; a high blood pressure side interlocking mechanism for interlocking the rotating basic shaft and the high blood pressure side indicator needle; a low blood pressure side interlocking mechanism for interlocking the rotating basic shaft and the low blood pressure side indicator needle; a high blood pressure side interlock releasing mechanism for releasing the interlocking state of the high blood pressure side interlocking mechanism based on the systolic blood pressure indication from the systolic blood pressure indicating unit; and a low blood pressure side interlock releasing mechanism for releasing the interlocking state of the low blood pressure side interlocking mechanism based on the diastolic blood pressure indication from the diastolic blood pressure indicating unit.

(8) The blood pressure level display device according to the foregoing (7), wherein: the high blood pressure side interlocking mechanism includes high blood pressure side one-way engaging means for interlocking the high blood pressure side indicator needle and the rotating basic shaft when the rotating basic shaft relatively rotates to higher blood pressures with respect to the high blood pressure side indicator needle, and high blood pressure side biasing means for biasing the high blood pressure side indicator needle to lower blood pressures rotationally against rotation of the high blood pressure side indicator needle to higher blood pressures; the low blood pressure side interlocking mechanism includes low blood pressure side one-way engaging means for interlocking the low blood pressure side indicator needle and the rotating basic shaft when the rotating basic shaft relatively rotates to higher blood pressures with respect to the low blood pressure side indicator needle, and low blood pressure side biasing means for biasing the low blood pressure side indicator needle to lower blood pressures rotationally against rotation of the low blood pressure side indicator needle to higher blood pressures; the high blood pressure side interlock releasing mechanism includes high blood pressure side braking means for coming into engagement with the high blood pressure side indicator needle to stop rotation of the high blood pressure side indicator needle; and the low blood pressure side interlock releasing mechanism includes low blood pressure side braking means for coming into engagement with the low blood pressure side indicator needle to stop rotation of the low blood pressure side indicator needle.

(9) The blood pressure level display device according to the foregoing (7) or (8), including a reset mechanism for releasing the interlocking states of the high blood pressure side interlocking mechanism and the low blood pressure side interlocking mechanism simultaneously, and rotating the high blood pressure side indicator needle and the low blood pressure indicator needle to their initial positions.

(10) The blood pressure level display device according to the foregoing (9), wherein the reset mechanism includes axial moving means for moving the rotating basic shaft axially relative to the high blood pressure side interlocking mechanism and the low blood pressure side interlocking mechanism and thereby releasing it forcibly when the rotating basic shaft lies near a zero point.

(11) A blood pressure measuring apparatus for measuring a blood pressure level of a subject to be measured, the apparatus including: the blood pressure level display device according to any one of the foregoing (1) to (10); and pressure control means for applying and reducing pressure to the subject to be measured, wherein the blood pressure level display device further includes motion converting means for converting a value of pressure the pressure control means applies to the subject to be measured into a rotational motion, and using the rotational motion as a power source for rotating the high blood pressure side indicator needle and the low blood pressure side indicator needle.

(12) A blood pressure level display method of using a blood pressure display panel provided with a circumferentially-calibrated scale intended for blood pressure levels, and rotating an indicator needle for displaying a blood pressure level so that the blood pressure level is visually identifiable from the scale indicated by the indicator needle, the method including: a systolic blood pressure indicating step of providing a systolic blood pressure indication based on a state of systolic blood pressure; a diastolic blood pressure indicating step of providing a diastolic blood pressure indication based on a state of diastolic blood pressure; a systolic blood pressure display step of rotating a high blood pressure side indicator needle in conjunction with a state of pressure applied to a subject to be measured, and stopping the high blood pressure side indicator needle based on the systolic blood pressure indication from the systolic blood pressure indicating unit, thereby displaying at rest a systolic blood pressure level on the scale pointed by the high blood pressure side indicator needle; and a diastolic blood pressure display step of rotating a low blood pressure side indicator needle in conjunction with the state of pressure applied to the subject to be measured, and stopping the low blood pressure side indicator needle based on the diastolic blood pressure indication from the diastolic blood pressure indicating unit, thereby displaying at rest a diastolic blood pressure level on the scale pointed by the low blood pressure side indicator needle.

### EFFECT OF THE INVENTION

According to the present invention, it is possible to improve the convenience when measuring blood pressure, so that measurements can be analyzed and examined in a short time.

### BEST MODE FOR CARRYING OUT THE INVENTION

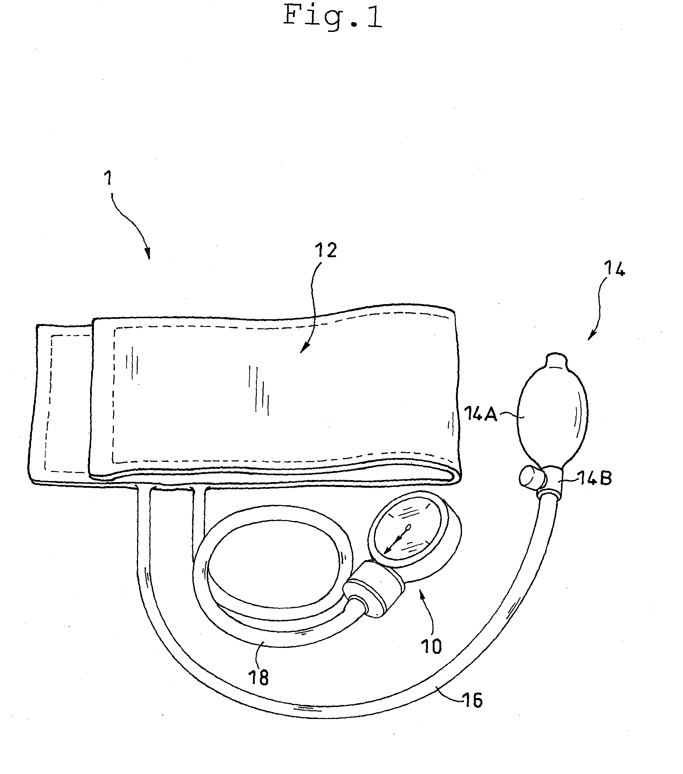

Fig. 1 shows the general configuration of a blood pressure measuring apparatus 1 according to a first embodiment of the present invention. This blood pressure measuring apparatus 1 includes a blood pressure level display device 10, a cuff 12, a pump 14, a supply tube 16, and a measurement tube 18. The cuff 12 is a pouched member made of rubber, synthetic resin, or the like, and inflates when air is introduced to the inside. This cuff 12 has the function of inflating itself to press a vessel for ischemia when tightly wrapped around the vicinity of a person's upper arm. The pump 14 is connected to the cuff 12 through the supply tube 16 made of rubber, and supplies air to the cuff 12 to increase the cuff pressure. Specifically, the pump 14 includes a pump part 14A of spherical shape, having an air-reserving space inside, and a pressure adjusting mechanism 14B for releasing the pressure inside the supply tube 16 by opening and closing its valve. When the pump part 14A is manually contracted, the inside air is supplied through the supply tube 16 into the cuff 12 to increase the cuff pressure. The cuff pressure once increased is maintained until the pressure adjusting mechanism 14B is opened. When this pressure adjusting mechanism 14B is opened, the air inside the cuff 12 is exhausted to lower the cuff pressure. The measurement tube 18 connects the cuff 12 and the blood pressure level display device 10.

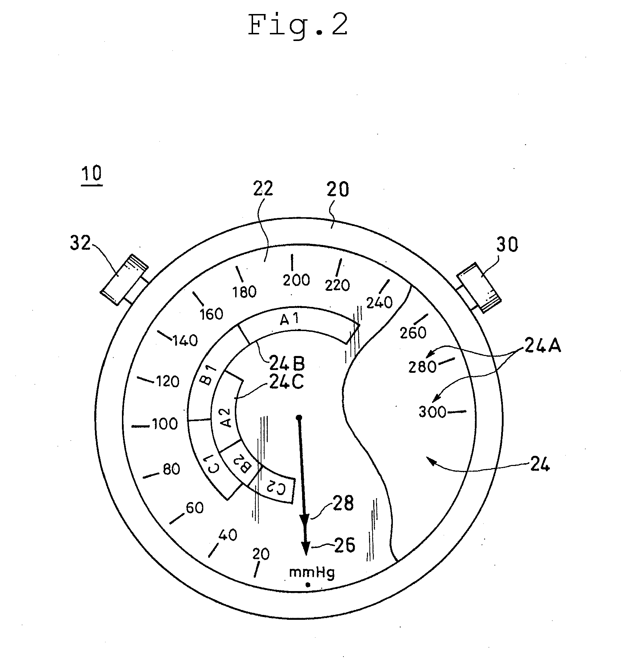

Fig. 2 is a top view of the blood pressure level display device 10. This blood pressure level display device 10 includes: an outer casing 20 of cylindrical shape; a transparent resin cover 22 which is arranged on the top of the outer casing 20; a blood pressure display panel 24 which is arranged inside and in parallel with the transparent resin cover 22; a high blood pressure side indicator needle 26 which rotates and stops on this blood pressure display panel 24 to display a systolic blood pressure level at rest; and a low blood pressure side indicator needle 28 which rotates and stops on the blood pressure display panel 24 to display a diastolic blood pressure level at rest. The lengths of the two indicator needles are set so that the high blood pressure indicator needle 26 is a long hand and the low blood pressure indicator needle 28 is a short hand.

The blood pressure display panel 24 has a circumferentially-calibrated scale 24A intended for blood pressure levels. Blood pressure levels can be checked visually from numerical values on the scale 24A indicated by the indicator needles 26 and 28. In this instance, the scale 24A is calibrated in units of mmHg (millimeters of mercury), with markings at every 20 mmHg in the range of 0 mmHg to 300 mmHg, within which range blood pressure levels can be measured.

The blood pressure display panel 24 also has a high blood pressure side numerical area 24B and a low blood pressure side numerical area 24C which are displayed along the scale 24A. The high blood pressure side numerical area 24B is a scale for determining the significance of the systolic blood pressure level, and is sectioned into three types including a high area A1, a middle area B1, and a low area C1 in respective different colors which are not shown in particular. These three types of sectioning including the high area A1, the middle area B1, and the low area C1 are established based on the WHO's high blood pressure classifications. For example, the low area C1 is a normal area, ranging from 120 mmHg to 130 mmHg exclusive. The middle area B1 is a border area, ranging from 130 mmHg to 139 mmHg inclusive. The high area A1 is a hypertension area, ranging from 140 mmHg to above. Consequently, the result (significance) of the high blood pressure level can be determined visually depending on which of the high area A1, the middle area B1, and the low area C1 the high blood pressure side indicator needle 26 stops at. Although not shown in particular, the high area A1 (hypertension area) is preferably subdivided like stage 1 (140 mmHg to 159 mmHg), stage 2 (160 mmHg to 179 mmHg), and stage 3 (180 mmHg and above) for more detailed understanding.

Similarly, the low blood pressure side numerical area 24C is a scale for determining the significance of the diastolic blood pressure level, and is sectioned into three types including a high area A2, a middle area B2, and a low area C2 in respective different colors. Consequently, the result (significance) of the low blood pressure level can be determined visually depending on which of the high area A2, the middle area B2, and the low area C2 the low blood pressure side indicator needle 28 stops at.

For example, the high area A1 means that the systolic blood pressure is too high. The middle area B1 means that the systolic blood pressure is in the border range. The low area C1 means that the blood pressure level is normal. Moreover, the high area A2 means that the diastolic blood pressure is too high. The middle area B2 means that the diastolic blood pressure is in the border range. The low area C2 means that the diastolic blood pressure level is normal. Further, it is also desirable, though not shown in particular, that the tip of the high blood pressure side indicator needle 26 be adjusted to fall within the range of the high blood pressure side numerical area 24B, and the tip of the low blood pressure side indicator needle 28 be adjusted within the range of the low blood pressure side numerical area 24C. While the present embodiment deals with the case where the high blood pressure side numerical area 24B and the low blood pressure side numerical area 24C are sectioned into three types, they may be sectioned into four or more types depending on the intended use.

A stop button 30 and a reset button 32 are arranged on the periphery of the outer casing 20. When a measurement operator manually performs a first button pressing operation on the stop button 30, it follows that this stop button 30 functions as a systolic blood pressure indicating unit to provide a systolic blood pressure indication. When the systolic blood pressure indication is provided, the high blood pressure side indicator needle 26 stops to display the systolic blood pressure level at rest. Moreover, when a second button pressing action is performed in succession to the first button pressing operation, it follows that a diastolic blood pressure indicating unit provides a diastolic blood pressure indication. When the diastolic blood pressure indication is provided, the low blood pressure side indicator needle 28 stops to display the diastolic blood pressure level at rest.

When the reset button 32 is pressed, a reset mechanism is activated so that the high blood pressure side indicator needle 26 and the low blood pressure side indicator needle 28 at rest are rotated to 0 mmHg and reset into their initial states.

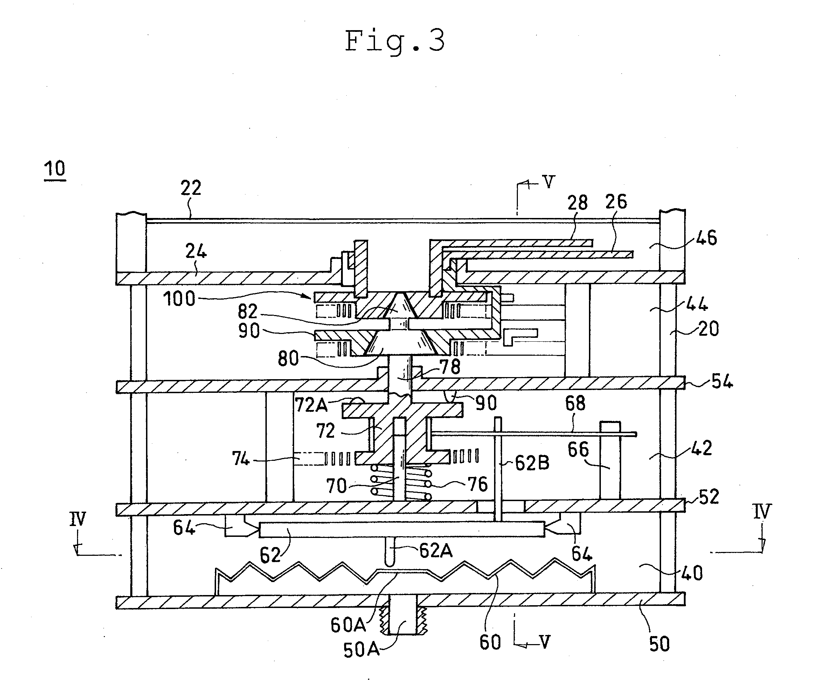

Fig. 3 is a cross-sectional view showing the internal configuration of the blood pressure level display device 10.

The outer casing 20 of cylindrical shape has a four-level structure inside, including a bottom layer 40, an intermediate layer 42, an upper layer 44, and a display layer 46 in order in the axial direction of the cylinder. It includes a base plate 50 which forms the bottom layer 40, a first partition plate 52 which partitions the bottom layer 40 and the intermediate layer 42, and a second partition plate 54 which partitions the intermediate layer 42 and the upper layer 44. It should be appreciated that the upper layer 44 and the display layer 46 are partitioned by the blood pressure display panel 24. The base plate 50 has an inlet port 50A for introducing compressed air from the cuff 12 to the inside through the measurement tube 18.

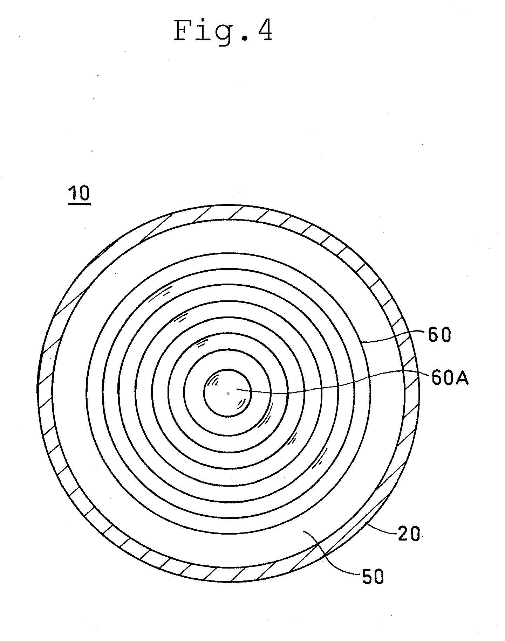

As shown in Fig. 4, a diaphragm 60 is integrally formed on the base plate 50 in the bottom layer 40.
This diaphragm 60 is formed by folding a circular plate member made of a spring material such as phosphor bronze, aluminum, or stainless steel along the circumferential direction alternately into an accordion-like configuration, and fixing it to the base plate 50. Consequently, when air is introduced into the diaphragm 60 through the inlet port 50A, the diaphragm 60 is inflated by the pressure of the air so that a detection area 60A formed in the center rises up. This rising distance is designed to be proportional to the internal pressure of the diaphragm 60, and this rising distance can be detected to detect a change in the cuff pressure.

Returning to Fig. 3, a conversion shaft 62 is rotatably held by the first partition plate 52 in the bottom layer 40.
Specifically, two needle-like holding pins 64 are fixed to the first partition plate 52, and the respective tips of these two holding pins 64 hold the ends of the conversion shaft 62.

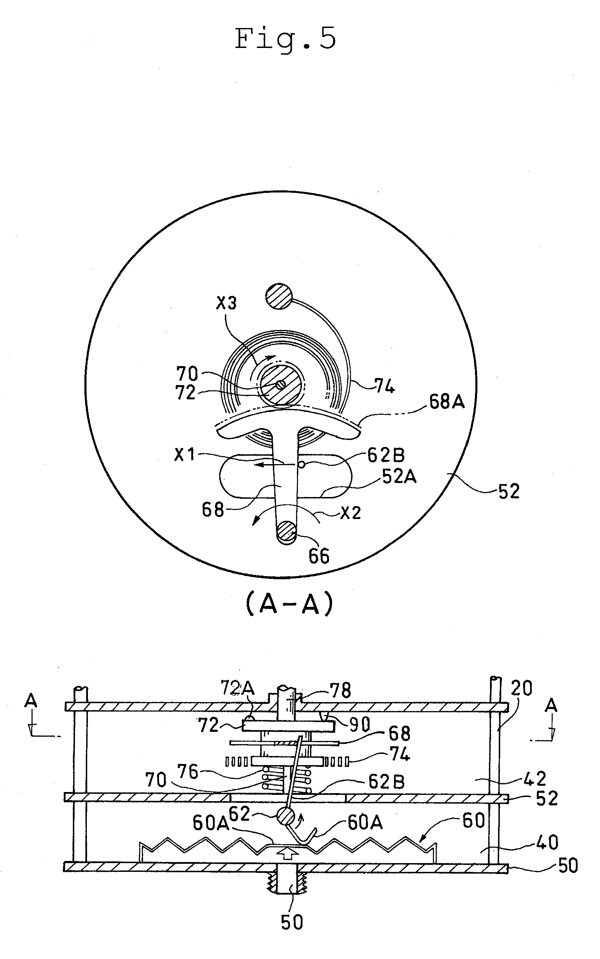

As shown in Fig. 5, the conversion shaft 62 has a first projection 62A which makes contact with the detection area 60A of the diaphragm 60, and a second projection 62B which penetrates through an opening 52A formed in the first partition layer 52 and protrudes into the intermediate layer 42. Consequently, when the detection area 60A of the diaphragm 60 rises up, the first projection 62A is pushed up to rotate the conversion shaft 62, and the second projection 62B lying in the intermediate layer 42 swings in conjunction with this rotation.

A support 66 is formed on the first partition plate 52 in the intermediate layer 42, and this support 66 holds a swinging partial gear 68. More specifically, this swinging partial gear 68 is rotatably held at one end by the support 66 so that it can swing in the direction of the plane of the first partition plate 52. In addition, a rack gear 68A is formed on the other end of the swinging partial gear 68 along a partial arc about the support 66. This swinging partial gear 68 is in contact with the second projection 62B. With this structure, when the second projection 62 moves in the direction of the arrow X1 (i.e., in the direction of swinging), the swinging partial gear 68 swings in the direction of the arrow X2 as biased by this second projection 62.

A slide shaft 70 is fixed to the center of the first partition plate 52 in the intermediate layer 42 so as to be coaxial with the outer casing 20. A slide gear 72 is rotatably and axially slidably mounted on this slide shaft 70. This slide gear 72 is in mesh with the rack gear 68A of the swinging partial gear 68. When the swinging partial gear 68 swings in the direction of the arrow X2, the slide gear 72 therefore rotates in the direction of the arrow X3. Meanwhile, the slide gear 72 is provided with a coil spring 74, which is biased in a direction opposite to the direction of driving X3 of the swinging partial gear 68. Consequently, the diaphragm 60 contracts and returns to its initial state. As a result, when the second projection 62B returns in the direction opposite to the arrow X1, the slide gear 72 actively rotates in the direction opposite to the arrow X3 with this coil spring 74 as a power source, thereby pushing the swinging partial gear 68 back to its original state. In this way, the movement of the diaphragm 60 and the rotation of the slide gear 72 are always interlocked with each other.

A spring 76 is mounted between the slide gear 72 and the first partition plate 52. This spring 76 biases the slide gear 72 upward (toward the upper layer 44) all the time. A rotating basic shaft 78 is integrally and coaxially formed on the top of this slide gear 70 (the side facing toward the upper layer 44). This rotating basic shaft 78 is thus also biased upward by the spring 76. Besides, a reset projection 72A is formed on the top of the slide gear 72 (the side facing toward the second partition plate 54) so as to be capable of engagement with a reset cam 90 which is formed on the second partition plate 54 in the intermediate layer 42. Note that the rotating basic shaft 78 protrudes into the upper layer 44 through an opening formed in the center of the second partition plate 54.

Returning to Fig. 3, a first engaging member 80 and a second engaging member 82 are integrally and coaxially formed on the top end of the rotating basic shaft 78 which protrudes into the upper layer 44. The first and second engaging members 80 and 82 have the shapes of partial cones, and are configured to rotate with the rotating basic shaft 78 (i.e., the slide gear 72). This second engaging member 80 is given a maximum diameter smaller than the minimum diameter of the first engaging member 80. This configuration is intended to facilitate assembly and also avoid interference when the two engaging members move in the axial directions.

A first driver 90 is capable of engagement with the first engaging member 80. A second driver 100 is capable of engagement with the second engaging member 82. As a result, when the first and second engaging members 80 and 82 rotate, the first driver 90 and the second driver 100 also rotate in conjunction with the same. More specifically, the tilt surfaces on the outer peripheries of the first and second engaging members 80 and 82 and the tilt surfaces on the inner peripheries of the first driver 90 and the second driver 100 make contact with each other so that they are integrated by the frictional forces therebetween.

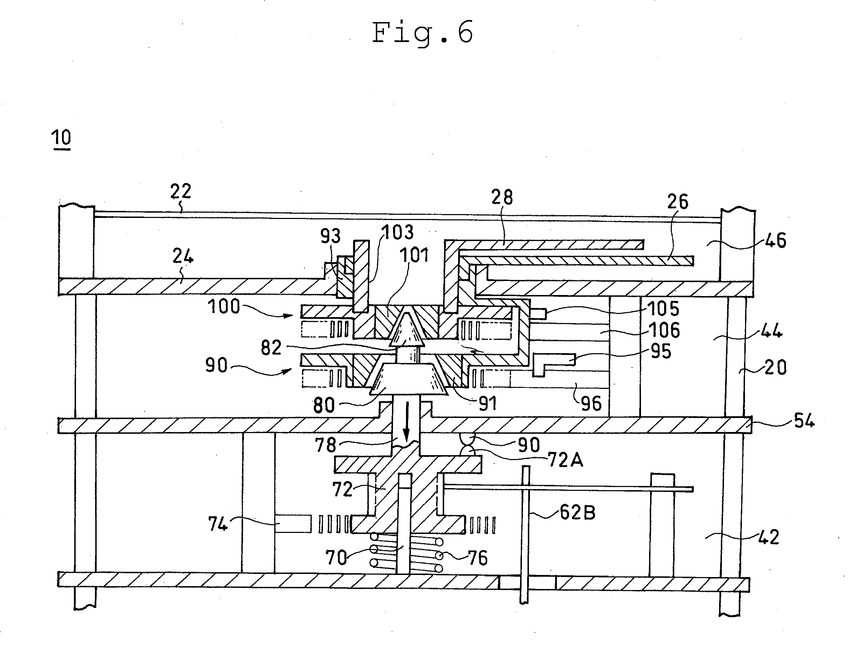

As shown in Fig. 6, when the slide gear 72 rotates to bring the reset cam 90 and the reset projection 72A into engagement, this reset cam 90 pushes down the reset projection 72A so that the reset gear 72, the rotating basic shaft 78, the first engaging member 80, and the second engaging member 82 are all pressed down against the biasing force from the spring 76. This detaches the first engaging member 80 and the second engaging member 82 from the first driver 90 and the second driver 100, thereby releasing the engagement therebetween. It should be appreciated that the reset cam 90 and the reset projection 72A are configured to come into engagement when the pressure inside the diaphragm 60 reaches an initial state (reset state). That is, when the diaphragm 60 is inflated and the rotating basic shaft 78 starts rotating, the cam engagement is released to lift the rotating basic shaft 78, whereby the first driver 90 and the second driver 100 are connected with the first engaging member 80 and the second engaging member 82 for integral rotation. When the diaphragm 60 contracts and the rotating basic shaft 78 rotates reversely back to the initial position, on the other hand, the cam makes an engagement to release the connection of the first driver 90 and the second driver 100 with the first engaging member 80 and the second engaging member 82.

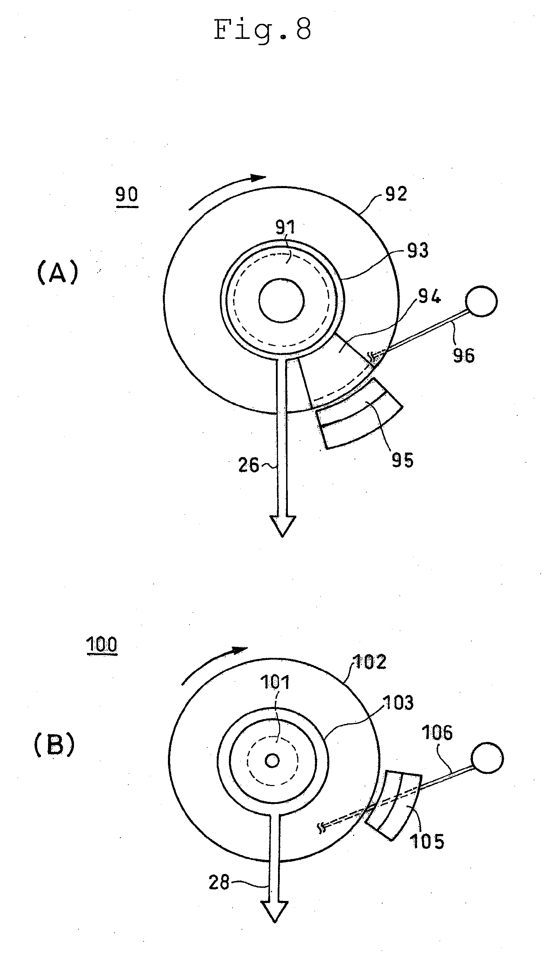

As shown in Figs. 6 to 8, the first driver 90 arranged in the upper layer 44 includes: a first bearing 91 which is capable of engagement with the first engaging member 80; a first brake ring 92 which is integrated with an outer ring of this first bearing 91; a first holding cylinder 92 of cylindrical shape, which is arranged at a predetermined distance from the first brake ring 92 and rotatably holds the high blood pressure side indicator needle 26; and an interlocking beam 94 which connects this first holding cylinder 93 and the first brake ring 92. The first bearing 91 is of one-way rotation type (such as a ratchet structure). If the first engaging member 80 starts to rotate relatively in the direction of increase of the blood pressure level with reference to the first brake ring 92, the inner and outer rings make an integral rotation to achieve interlocking therebetween. On the other hand, if the first engaging member 80 starts to rotate relatively in the direction of decrease of the blood pressure level with respect to the first brake ring 92, the first engaging member 80 can rotate alone by priority.

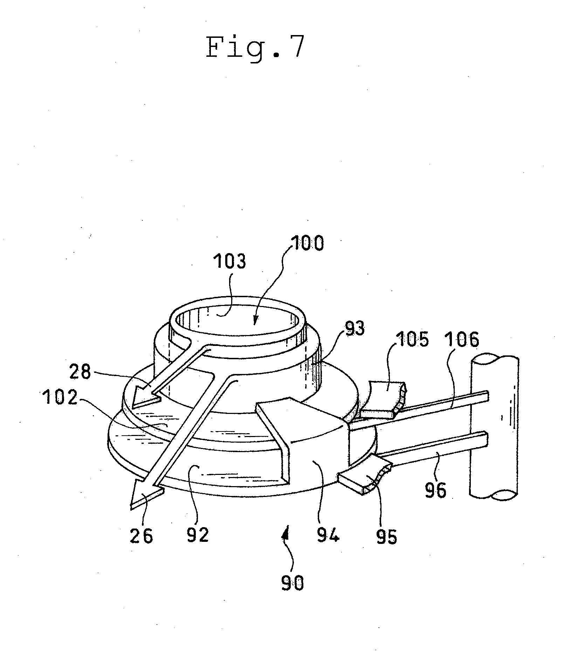

The outer periphery of the first brake ring 92 is configured to make contact with the first brake pad 95 when necessary, so that the frictional force stops the rotation of this first brake ring 92 forcibly. This first brake ring 92 is also coupled with a first coil spring 96, which always biases the first brake ring 92 to rotate in the direction of decrease of the blood pressure level. The first holding cylinder 93 is rotatably held by an opening formed in the blood pressure display panel 24. The interlocking beam 94 is a member having a radial section of inverted L shape, and is connected to the outer periphery of the first holding cylinder 93 at one end and connected to near the outer rim of the brake ring 92 at the other end. This allows integral rotation of the first brake ring 92 and the first holding cylinder 93 while maintaining a gap capable of accommodating the second driver 100 between the first brake ring 92 and the first holding cylinder 93. As shown in Fig. 7 and the like, the interlocking beam 94 is given a circumferential width not across the entire circumference of the first brake ring 92 but within a partial angular range of approximately 20° or so, in order to avoid interference with the braking operation of the first driver 100 and the coil springs to be described later when this first driver 90 rotates.

The second driver 100 includes: a second bearing 101 which is capable of engagement with the second engaging member 82; a second brake ring 102 which is integrated with an outer ring of this second bearing 101; and a second holding cylinder 103 of cylindrical shape, which is coaxially and integrally arranged on the second brake ring 102 and rotatably holds the low blood pressure side indicator needle 28. The second bearing 101 is of one-way type. If the second engaging member 82 starts to rotate relatively in the direction of increase of the blood pressure level with reference to the second brake ring 102, the inner and outer rings make an integral rotation to achieve interlocking therebetween. On the other hand, if the second engaging member 82 starts to rotate relatively in the direction of decrease of the blood pressure level with respect to the second brake ring 102, the second engaging member 82 can rotate alone by priority.

A second brake pad 105 is arranged on the outer periphery of the second brake ring 102. The two can make contact when necessary, thereby stopping the rotation of this second brake ring 102. This second brake ring 102 is also coupled with a second coil spring 106, which always biases the second brake ring 102 to rotate in the direction of decrease of the blood pressure level. The second holding cylinder 103 is held by the inner periphery of the first holding cylinder 93 of the first driver 90 so as to be capable of relative rotation.

Next, an example of operation of this blood pressure measuring apparatus 1 will be described. When the cuff pressure is zero, as shown in Fig. 6, the first engaging member 80 and the second engaging member 82 are released from the first driver 90 and the second driver 100, or in a so-called initial state, in the blood pressure level display device 10. When the cuff 12 is wrapped around an examinee's upper arm and air is supplied from the pump 14 into the cuff 12 to increase the cuff pressure, the diaphragm 60 of the blood pressure display device 10 is inflated to swing the swinging partial gear 68 via the conversion shaft, and the slide gear 72 starts rotating. At that point in time, the engagement between the reset cam 90 and the reset projection 72A is released. The slide gear 72 is pushed to slide upward by the spring 76, and the first engaging member 80 and the second engaging member 82 are connected with the first driver 90 and the second driver 100. As a result, the inner and outer rings of the first and second bearings 91 and 101 of ratchet structure come into engagement so that the entire members start to rotate integrally.

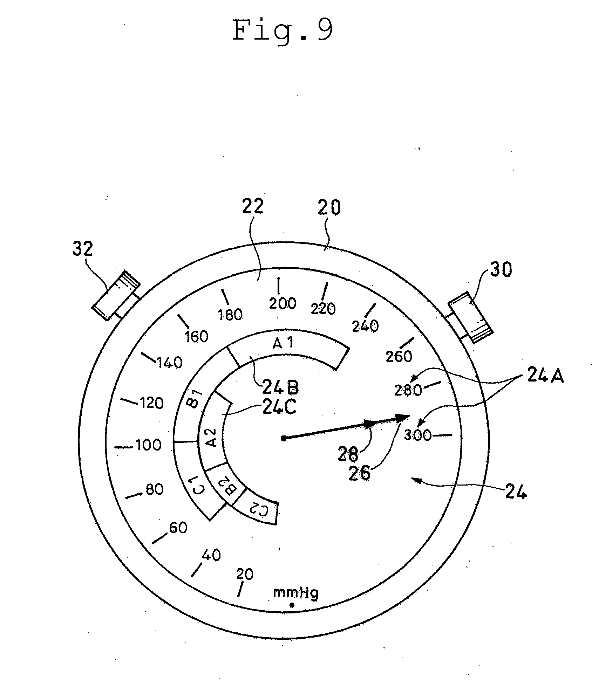

As shown in Fig. 9, this rotates the high blood pressure side indicator needle 26 and the low blood pressure side indicator needle 28 simultaneously, so that the two indicator needles 26 and 28 display the same cuff pressure. After the cuff pressure is increased to a desired level, the pressure adjustment mechanism 14B arranged on the pump 14 is operated to lower the cuff pressure gradually. In conjunction with this, the rotating basic shaft 78 rotates in the decreasing direction. Here, the first driver 90 and the second driver 100 are biased to rotate in the decreasing direction by the first coil spring 96 and the second coil spring 106, respectively, and thus return in the decreasing direction ahead of the rotation of the rotating basic shaft 78 in the decreasing direction. In relative terms, it follows that the rotating basic shaft 78 rotates in the increasing direction with respect to the high blood pressure side indicator needle 26 and the low blood pressure side indicator needle 28. The engagement of the ratchet structures of the first and second bearings 91 and 101 is therefore not released, and the high blood pressure side indicator needle 26 and the low blood pressure side indicator needle 28 also make a reverse rotation toward the pressure-decreasing side simultaneously with the rotating basic shaft 78.

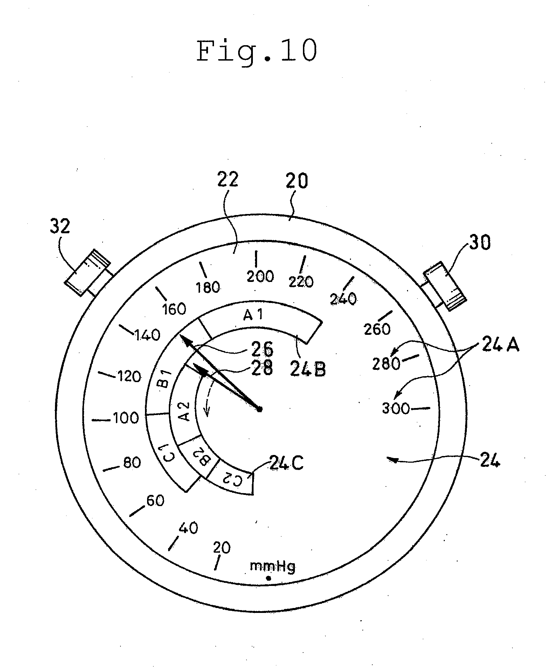

When a pulse sound is heard from the upper arm and it is recognized as the timing of the systolic blood pressure, the examiner presses the stop button 30. As a result, a moving mechanism which is not shown in particular moves the first brake pad 95 toward and into contact with the first brake ring 92, so that the rotation of the first driver 90 stops due to the frictional force. When the rotation of the first brake ring 92 is stopped, it follows that the rotating basic shaft 78 relatively rotates in the decreasing direction with respect to the first driver 90. This releases the engagement of the ratchet mechanism in the first bearing 91, thereby allowing the first engaging member 80 alone to continue rotating in the decreasing direction. In consequence, as shown in Fig. 10, the high blood pressure side indicator needle 26 stops at the timing of the systolic blood pressure while the low blood pressure side indicator needle 28 continues rotating in the decreasing direction in conjunction with the actual cuff pressure.

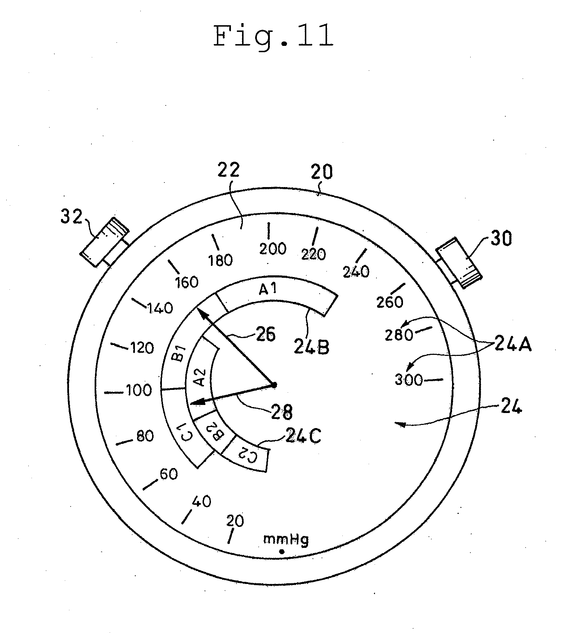

Subsequently, when pulse sounds are no longer heard from the upper arm and it is recognized as the timing of the diastolic blood pressure, the examiner presses the stop button 30 again. As a result, the second brake pad 105 moves toward and into contact with the second brake ring 102, and the rotation of the second driver 100 stops due to the frictional force. When the rotation of the second brake ring 102 is stopped, the engagement of the ratchet mechanism in the second bearing 101 is released so that the second engaging member 82 alone continues rotating in the decreasing direction. Eventually, as shown in Fig. 11, the low blood pressure side indicator needle 28 stops at the timing of the diastolic blood pressure. Although not shown in particular, the rotating basic shaft 78 and the like continue rotating in the decreasing direction in conjunction with the actual cuff pressure.

As a result of the foregoing, the systolic blood pressure level and the diastolic blood pressure level can be grasped visually from the high blood pressure side indicator needle 26 and the low blood pressure side indicator needle 28 at rest.

After the completion of the measurement, the pressure adjusting mechanism 14B of the pump 14 is operated to bring the cuff pressure into an open state (the same condition as the surroundings) completely, thereby restoring the diaphragm 60 to its initial state. When the slide gear 72 returns to near zero (initial state) in conjunction with this, the reset cam 90 and the reset projection 72A come into engagement to slide the slide gear 72 downward, whereby the first engaging member 80 and the second engaging member 82 are released from the first driver 90 and the second driver 100 as previously shown in Fig. 6. When the examiner presses the reset button 32 in this state, the first brake pad 95 and the second brake pad 105 retreat at the same time, thereby releasing both the first brake ring 92 and the second brake ring 102. As a result, because of the biasing forces from the first coil spring 96 and the second coil spring 106, the first driver 90 and the second river 100 instantaneously return to zero (initial state) and are reset to the state of Fig. 2.

According to the blood pressure measuring apparatus 1 of the present embodiment, the high blood pressure side indicator needle 26 and the low blood pressure side indicator needle 28 are both stopped in the blood pressure level display device 10 so that they can be checked after the completion of the measurement. It is therefore possible to avoid measurement errors. This also makes it possible to grasp the blood pressure conditions of the examinee immediately (visually) from the stopped angles of the needles. Specifically, the stop button 30 has only to be pressed to stop the first driver 90 and the second driver 100 independently with the frictional forces from the first brake pad 95 and the second brake pad 105, so that the systolic blood pressure and the diastolic blood pressure can be displayed at rest. Moreover, the one-way engagement structures (here, ratchet structures) of the first and second bearings 91 and 101 prevent the braking operations from hampering the rotation of the rotating basic shaft 82 (the rotation in the direction of decrease of the pressure level).

Furthermore, the blood pressure display panel 24 is provided with the high blood pressure side numerical area 24B and the low blood pressure side numerical area 24C which are displayed along the scale 24A. This makes it possible to grasp the blood pressure conditions intuitively from the areas where the high blood pressure side indicator needle 26 and the low blood pressure side indicator needle 28 stop at. In particular, since the high blood pressure side and low blood pressure side numerical areas 24B and 24C are arranged in an adjacent state, it is possible to grasp the conditions on the respective high blood pressure side and low blood pressure side simultaneously, and examine the correlation therebetween as well. This can significantly improve the convenience when analyzing the blood pressure conditions. In addition, the high blood pressure side indicator needle 26 is formed as a long hand and the low blood pressure side indicator needle 28 as a short hand, and the high blood pressure side numerical area 24B is located radially outside and the low blood pressure side numerical area 24C radially inside. This provides an easy-to-view state as a whole. Besides, the numerical areas 24B and 24C are sectioned into three types of areas in respective different colors each, thereby facilitating an analysis and examination on the high blood pressure level and the low blood pressure level.

It should be appreciated that after the completion of the measurement, the cuff pressure can be reduced to zero to release the engagement between the rotating basic shaft 78 and the first and second drivers 90 and 100 automatically by force. The high blood pressure side indicator needle 26 and the low blood pressure side indicator needle 28 can thus be easily restored to their initial states by only pressing the reset button 32. This makes it possible to facilitate the operations while achieving a sophisticated display mode.

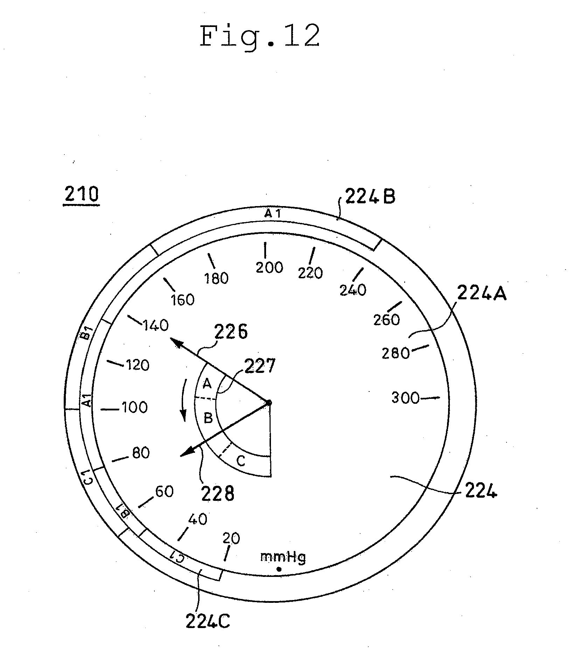

Next, a blood pressure level display device 210 according to a second embodiment of the present invention will be described with reference to Fig. 12. Note that the internal structure and the like of this blood pressure level display device 210 are generally the same as those of the blood pressure level display device 10 according to the first embodiment, and thus will not be shown in the drawings. Besides, components and members shown in Fig. 12 that are identical or similar to those of the blood pressure level display device 10 will be designated by reference numerals having the same last two digits as in the first embodiment, and a detailed description thereof will be omitted.

A high blood pressure side numerical area 224B and a low blood pressure side numerical area 224C are displayed radially outside a scale 224A of a blood pressure display panel 224. More specifically, these areas 224B and 224C are formed on the end face of an outer casing 220. Moreover, a blood pressure difference display plate 226A having the shape of a sector about the rotation axis is fixed to a high blood pressure side indicator needle 226, or long hand, so that it rotates with the high blood pressure side indicator needle 226.

This blood pressure difference display plate 226 is provided with a scale or an area display. The area display formed there includes an A area, a B area, and a C area in different colors. This blood pressure difference display plate 226 is intended to grasp an angular difference between the high blood pressure side indicator needle 226 and the low blood pressure side indicator needle 228, i.e., a difference between the systolic blood pressure and the diastolic blood pressure visually. This allows instant understanding of a relative analysis between the systolic blood pressure and the diastolic blood pressure, such as: when the low blood pressure side indicator needle 228 lies in the A area, the blood pressure difference is determined to be too small; when in the B area, the blood pressure difference is moderate; and when in the C area, the blood pressure difference is too large.

According to the blood pressure level display device 210 of this second embodiment, it is therefore possible to perform independent analyses on the systolic blood pressure and the diastolic blood pressure visually by using the high blood pressure side numerical area 224B and the low blood pressure side numerical area 224C, while performing a relative analysis therebetween at the same time. It should be appreciated that this blood pressure difference display plate 226 may be configured to move with the short hand, or the low blood pressure side indicator needle 228.

Up to this point, the first and second embodiments have shown some examples of embodiment of the present invention. The systolic blood pressure indicating unit and the diastolic blood pressure indicating unit, as employed in the present invention, correspond in part to the stop button 30 of the blood pressure measuring apparatus 1 when adopted in the first embodiment. That is, the stop button 30 is a part of the systolic blood pressure indicating unit and the diastolic blood pressure indicating unit, being shared between the two indicating units. While in the present embodiments the indications of the timing of the systolic and diastolic blood pressures are given manually, the present invention is not limited thereto. The systolic and diastolic blood pressure indications may be output automatically at timing that is mechanically or electronically measured by automatic measurement.

Moreover, the high blood pressure side interlocking mechanism, as employed in the present invention, corresponds to the first engaging member 80, the first driver 90, and the like when adopted in the first embodiment. Specifically, these members have the function of interlocking the rotation of the rotating basic shaft 78 and the rotation of the high blood pressure side indicator needle 26. Similarly, the low blood pressure side interlocking mechanism corresponds to the second engaging member 2, the second driver 100, and the like, having the function of interlocking the rotation of the rotating basic shaft 78 and the rotation of the low blood pressure side indicator needle 28. Moreover, the one-way engaging means in the high blood pressure side and low blood pressure side interlocking mechanisms of the present invention corresponds to the first and second bearings 91 and 101 having a ratchet mechanism when adopted in the first embodiment. It should be appreciated that the one-way engaging means is not limited to the ratchet mechanisms. Any other mechanism may be used as long as it has the structure of establishing engagement between its inside and outside when the inside and outside have a difference in rotational phase in one direction, and releasing the engagement when they have a difference in rotational phase in the other direction. Moreover, the biasing means of the present invention corresponds to the first and second coil springs 96 and 106 when adopted in the first embodiment.

Now, the motion converting means of the present invention corresponds to the diaphragm 60, the conversion shaft 62, the swinging partial gear 68, the slide gear 72, and the like when adopted in the first embodiment. Moreover, the high blood pressure side interlock releasing mechanism, when adopted in the first embodiment, corresponds to the first bearing 91 having a ratchet mechanism, and the first brake ring 92, the first brake pad 95, and the like that function as braking means. It can release the interlocking state of the high blood pressure side interlocking mechanism based on the systolic blood pressure indication via the stop button 30. Similarly, the low blood pressure side interlock releasing mechanism of the present invention corresponds to the second bearing 101 having a ratchet mechanism, and the second brake ring 102, the second brake pad 105, and the like that function as braking means. It can release the interlocking state of the low blood pressure side interlocking mechanism based on the diastolic blood pressure indication via the stop button 30.

Moreover, the reset mechanism according to the present invention, when adopted in the first embodiment, corresponds to the reset button 32, the first and second brake pads 95 and 105, the first and second coil springs 96 and 106, etc. Furthermore, the axial moving means of the reset mechanism according to the present invention corresponds to the reset cam 90 and the reset projection 72A in the present embodiments.

It should be appreciated that the present embodiments have dealt with a method of measuring blood pressure where the cuff is wrapped around an upper arm. The present invention is not limited thereto, however, but may cover various other measuring methods such as pressing the vicinity of a wrist, finger, or the like and checking a pulse response for measurement. The present embodiments have also dealt only with the blood pressure level display devices where needles are actually rotated mechanically. The present invention is not limited thereto, however, but may also cover the cases where image display means such as a liquid crystal display is used to display similar situations as an image.

It is also understood that the present invention is not limited to the foregoing embodiments, and modifications may be made as appropriate without departing from the gist of the present invention.

### INDUSTRIAL APPLICABILITY

According to the present invention, it is possible to improve the convenience when measuring blood pressure, and allow an instant analysis and examination on the measurements. The present invention is applicable to various types of blood pressure measuring apparatuses.

### BRIEF DESCRIPTION OF THE DRAWINGS

Fig. 1 is a diagram showing the general configuration of a blood pressure measuring apparatus according to a first embodiment;
Fig. 2 is an enlarged plan view showing a blood pressure level display device of the blood pressure measuring apparatus;
Fig. 3 is an enlarged cross-sectional view showing the blood pressure level display device;
Fig. 4 is a sectional view taken along the line IV-IV of Fig. 3;
Fig. 5 is an enlarged partial cross-sectional view showing the blood pressure level display device;
Fig. 6 is an enlarged partial cross-sectional view showing the blood pressure level display device;
Fig. 7 is an enlarged perspective view showing first and second drivers of the blood pressure level display device;
Fig. 8 is an enlarged plan view showing (A) the first driver and (B) the second driver of the blood pressure level display device;
Fig. 9 is a plan view showing the movement of indicator needles of the blood pressure level display device in use;
Fig. 10 is a plan view showing the movement of the indicator needles of the blood pressure level display device in use;
Fig. 11 is a plan view showing the movement of the indicator needles of the blood pressure level display device in use; and
Fig. 12 is a plan view showing a blood pressure level display device according to a second embodiment.

### DESCRIPTION OF THE REFERENCE NUMERALS

- 1: blood pressure measuring apparatus
- 10, 210: blood pressure level display device
- 12: cuff
- 14: pump
- 20: outer casing
- 24, 224: blood pressure display panel 24
- 26, 226: high blood pressure side indicator needle
- 28, 228: low blood pressure side indicator needle
- 30: stop button
- 32: reset button
- 60: diaphragm
- 62: conversion shaft
- 68: swinging partial gear
- 70: slide shaft
- 72: slide gear
- 78: rotating basic shaft
- 80: first engaging member
- 82: second engaging member
- 90: first driver
- 91: first bearing
- 100: second driver
- 101: second bearing

## Claims

1. A blood pressure level display device having a blood pressure display panel provided with a circumferentially-calibrated scale intended for blood pressure levels, with respect to which an indicator needle for displaying a blood pressure level rotates so that the blood pressure level is visually identifiable from the scale indicated by the indicator needle, the device comprising:
a systolic blood pressure indicating unit for providing a systolic blood pressure indication based on a state of systolic blood pressure;
a diastolic blood pressure indicating unit for providing a diastolic blood pressure indication based on a state of diastolic blood pressure;
a high blood pressure side indicator needle which rotates in conjunction with a state of pressure applied to a subject to be measured, is allowed to stop based on the systolic blood pressure indication from the systolic blood pressure indicating unit, and displays at rest a systolic blood pressure level on the scale; and
a low blood pressure side indicator needle which rotates in conjunction with the state of pressure applied to the subject to be measured, is allowed to stop based on the diastolic blood pressure indication from the diastolic blood pressure indicating unit, and displays at rest a diastolic blood pressure level on the scale.

2. The blood pressure level display device according to claim 1, comprising:
a high blood pressure side numerical area for determining significance of the systolic blood pressure level pointed by the high blood pressure side indicator needle, being circumferentially arranged along the scale; and
a low blood pressure side numerical area for determining significance of the diastolic blood pressure level pointed by the low blood pressure side indicator needle, being circumferentially arranged along the scale, and
wherein a result of blood pressure measurement can be determined visually from a stop position of the high blood pressure side indicator needle in the high blood pressure side numerical area and a stop position of the low blood pressure side indicator needle in the low blood pressure side numerical area.

3. The blood pressure level display device according to claim 2, wherein the high blood pressure side numerical area and the low blood pressure side numerical area are each sectioned into at least three types of areas including a high area, a middle area, and a low area.

4. The blood pressure level display device according to claim 1, 2, or 3, wherein the high blood pressure side indicator needle is a long hand, and the low blood pressure side indicator needle is a short hand.

5. The blood pressure level display device according to claim 2, wherein the low blood pressure side numerical area is displayed radially inside the high blood pressure side numerical area so that a needlepoint of the high blood pressure side indicator needle, the long hand, falls within the high blood pressure side numerical area and a needlepoint of the low blood pressure side indicator needle, the short hand, falls within the low blood pressure side numerical area.

6. The blood pressure level display device according to any one of claims 1 to 5, comprising blood pressure difference display means fixed to either one of the high blood pressure side indicator needle and the low blood pressure side indicator needle, a blood pressure difference between the systolic blood pressure and the diastolic blood pressure being identifiable based on a relative angular difference between the high blood pressure side indicator needle and the low blood pressure side indicator needle at rest.

7. The blood pressure level display device according to any one of claims 1 to 6, comprising:
a rotating basic shaft for rotating in conjunction with the state of pressure applied to the subject to be measured;
a high blood pressure side interlocking mechanism for interlocking the rotating basic shaft and the high blood pressure side indicator needle;
a low blood pressure side interlocking mechanism for interlocking the rotating basic shaft and the low blood pressure side indicator needle;
a high blood pressure side interlock releasing mechanism for releasing the interlocking state of the high blood pressure side interlocking mechanism based on the systolic blood pressure indication from the systolic blood pressure indicating unit; and
a low blood pressure side interlock releasing mechanism for releasing the interlocking state of the low blood pressure side interlocking mechanism based on the diastolic blood pressure indication from the diastolic blood pressure indicating unit.

8. The blood pressure level display device according to claim 7, wherein:
the high blood pressure side interlocking mechanism includes
high blood pressure side one-way engaging means for interlocking the high blood pressure side indicator needle and the rotating basic shaft when the rotating basic shaft relatively rotates to higher blood pressures with respect to the high blood pressure side indicator needle, and
high blood pressure side biasing means for biasing the high blood pressure side indicator needle to lower blood pressures rotationally against rotation of the high blood pressure side indicator needle to higher blood pressures;
the low blood pressure side interlocking mechanism includes
low blood pressure side one-way engaging means for interlocking the low blood pressure side indicator needle and the rotating basic shaft when the rotating basic shaft relatively rotates to higher blood pressures with respect to the low blood pressure side indicator needle, and
low blood pressure side biasing means for biasing the low blood pressure side indicator needle to lower blood pressures rotationally against rotation of the low blood pressure side indicator needle to higher blood pressures;
the high blood pressure side interlock releasing mechanism includes high blood pressure side braking means for coming into engagement with the high blood pressure side indicator needle to stop rotation of the high blood pressure side indicator needle; and
the low blood pressure side interlock releasing mechanism includes low blood pressure side braking means for coming into engagement with the low blood pressure side indicator needle to stop rotation of the low blood pressure side indicator needle.

9. The blood pressure level display device according to claim 7 or 8, comprising a reset mechanism for releasing the interlocking states of the high blood pressure side interlocking mechanism and the low blood pressure side interlocking mechanism simultaneously, and rotating the high blood pressure side indicator needle and the low blood pressure indicator needle to their initial positions.

10. The blood pressure level display device according to claim 9, wherein the reset mechanism includes axial moving means for moving the rotating basic shaft axially relative to the high blood pressure side interlocking mechanism and the low blood pressure side interlocking mechanism and thereby releasing it forcibly when the rotating basic shaft lies near a zero point.

11. A blood pressure measuring apparatus for measuring a blood pressure level of a subject to be measured, the apparatus comprising:
the blood pressure level display device according to any one of claims 1 to 10; and
pressure control means for applying and reducing pressure to the subject to be measured,
wherein the blood pressure level display device further includes motion converting means for converting a value of pressure the pressure control means applies to the subject to be measured into a rotational motion, and using the rotational motion as a power source for rotating the high blood pressure side indicator needle and the low blood pressure side indicator needle.

12. A blood pressure level display method of using a blood pressure display panel provided with a circumferentially-calibrated scale intended for blood pressure levels, and rotating an indicator needle for displaying a blood pressure level so that the blood pressure level is visually identifiable from the scale indicated by the indicator needle, the method comprising:
a systolic blood pressure indicating step of providing a systolic blood pressure indication based on a state of systolic blood pressure;
a diastolic blood pressure indicating step of providing a diastolic blood pressure indication based on a state of diastolic blood pressure;
a systolic blood pressure display step of rotating a high blood pressure side indicator needle in conjunction with a state of pressure applied to a subject to be measured, and stopping the high blood pressure side indicator needle based on the systolic blood pressure indication from the systolic blood pressure indicating unit, thereby displaying at rest a systolic blood pressure level on the scale pointed by the high blood pressure side indicator needle; and
a diastolic blood pressure display step of rotating a low blood pressure side indicator needle in conjunction with the state of pressure applied to the subject to be measured, and stopping the low blood pressure side indicator needle based on the diastolic blood pressure indication from the diastolic blood pressure indicating unit, thereby displaying at rest a diastolic blood pressure level on the scale pointed by the low blood pressure side indicator needle.

## Amended claims

### Amended claims under Art. 19.1 PCT

1. A blood pressure level display device (10) having a blood pressure display panel (24) provided with a circumferentially-calibrated scale (24A) intended for blood pressure levels, with respect to which an indicator needle (26, 28) for displaying a blood pressure level rotates so that the blood pressure level is visually identifiable from the scale (24A) indicated by the indicator needle (26, 28), the device (10) **characterized by** comprising:
a systolic blood pressure indicating unit (30) for providing a systolic blood pressure indication based on a state of systolic blood pressure;
a diastolic blood pressure indicating unit (30) for providing a diastolic blood pressure indication based on a state of diastolic blood pressure;
a high blood pressure side indicator needle (26) which rotates in conjunction with a state of pressure applied to a subject to be measured, is allowed to stop based on the systolic blood pressure indication from the systolic blood pressure indicating unit (30), and displays at rest a systolic blood pressure level on the scale (24A); and
a low blood pressure side indicator needle (28) which rotates in conjunction with the state of pressure applied to the subject to be measured, is allowed to stop based on the diastolic blood pressure indication from the diastolic blood pressure indicating unit (30), and displays at rest a diastolic blood pressure level on the scale (24A).

2. The blood pressure level display device (10) according to claim 1, **characterized by** comprising:
a high blood pressure side numerical area (24B) for determining significance of the systolic blood pressure level pointed by the high blood pressure side indicator needle (26), being circumferentially arranged along the scale (24A); and
a low blood pressure side numerical area (24C) for determining significance of the diastolic blood pressure level pointed by the low blood pressure side indicator needle (28), being circumferentially arranged along the scale (24A), and
wherein a result of blood pressure measurement can be determined visually from a stop position of the high blood pressure side indicator needle (26) in the high blood pressure side numerical area (24B) and a stop position of the low blood pressure side indicator needle (28) in the low blood pressure side numerical area (24C).

3. The blood pressure level display device (10) according to claim 2, **characterized in that** the high blood pressure side numerical area (24B) and the low blood pressure side numerical area (24C) are each sectioned into at least three types of areas including a high area (A1, A2), a middle area (B1, B2), and a low area (C1, C2).

4. The blood pressure level display device (10) according to claim 1, 2, or 3, **characterized in that** the high blood pressure side indicator needle (26) is a long hand, and the low blood pressure side indicator needle (28) is a short hand.

5. The blood pressure level display device (10) according to claim 2, **characterized in that** the low blood pressure side numerical area (24C) is displayed radially inside the high blood pressure side numerical area (24B) so that a needlepoint of the high blood pressure side indicator needle (26), the long hand, falls within the high blood pressure side numerical area (24B) and a needlepoint of the low blood pressure side indicator needle (28), the short hand, falls within the low blood pressure side numerical area (24C).

6. The blood pressure level display device (10) according to any one of claims 1 to 5, **characterized by** comprising blood pressure difference display means (92, 95, 102, 105) fixed to either one of the high blood pressure side indicator needle (26) and the low blood pressure side indicator needle (28), a blood pressure difference between the systolic blood pressure and the diastolic blood pressure being identifiable based on a relative angular difference between the high blood pressure side indicator needle (26) and the low blood pressure side indicator needle (28) at rest.

7. The blood pressure level display device (10) according to any one of claims 1 to 6, **characterized by** comprising:
a rotating basic shaft (78) for rotating in conjunction with the state of pressure applied to the subject to be measured;
a high blood pressure side interlocking mechanism (80, 90) for interlocking the rotating basic shaft (78) and the high blood pressure side indicator needle (26);
a low blood pressure side interlocking mechanism (82, 100) for interlocking the rotating basic shaft (78) and the low blood pressure side indicator needle (28);
a high blood pressure side interlock releasing mechanism (91, 92, 95) for releasing the interlocking state of the high blood pressure side interlocking mechanism (80, 90) based on the systolic blood pressure indication from the systolic blood pressure indicating unit (30); and
a low blood pressure side interlock releasing mechanism (101, 102, 105) for releasing the interlocking state of the low blood pressure side interlocking mechanism (82, 100) based on the diastolic blood pressure indication from the diastolic blood pressure indicating unit (30).

8. The blood pressure level display device (10) according to claim 7, **characterized in that**:
the high blood pressure side interlocking mechanism (80, 90) includes
high blood pressure side one-way engaging means (91) for interlocking the high blood pressure side indicator needle (26) and the rotating basic shaft (78) when the rotating basic shaft (78) relatively rotates to higher blood pressures with respect to the high blood pressure side indicator needle (26), and
high blood pressure side biasing means (96) for biasing the high blood pressure side indicator needle (26) to lower blood pressures rotationally against rotation of the high blood pressure side indicator needle (26) to higher blood pressures;
the low blood pressure side interlocking mechanism (82, 100) includes
low blood pressure side one-way engaging means (101) for interlocking the low blood pressure side indicator needle (28) and the rotating basic shaft (78) when the rotating basic shaft (78) relatively rotates to higher blood pressures with respect to the low blood pressure side indicator needle (28), and
low blood pressure side biasing means (106) for biasing the low blood pressure side indicator needle (28) to lower blood pressures rotationally against rotation of the low blood pressure side indicator needle (28) to higher blood pressures;
the high blood pressure side interlock releasing mechanism (91, 92, 95) includes high blood pressure side braking means (92, 95) for coming into engagement with the high blood pressure side indicator needle (26) to stop rotation of the high blood pressure side indicator needle (26); and
the low blood pressure side interlock releasing mechanism (101, 102, 105) includes low blood pressure side braking means (102, 105) for coming into engagement with the low blood pressure side indicator needle (28) to stop rotation of the low blood pressure side indicator needle (28).

9. The blood pressure level display device (10) according to claim 7 or 8, **characterized by** comprising a reset mechanism (32, 95, 96, 105, 106) for releasing the interlocking states of the high blood pressure side interlocking mechanism (80, 90) and the low blood pressure side interlocking mechanism (82, 100) simultaneously, and rotating the high blood pressure side indicator needle (26) and the low blood pressure indicator needle (28) to their initial positions.

10. The blood pressure level display device (10) according to claim 9, **characterized in that** the reset mechanism (32, 95, 96, 105, 106) includes axial moving means (90, 72A) for moving the rotating basic shaft (78) axially relative to the high blood pressure side interlocking mechanism (80, 90) and the low blood pressure side interlocking mechanism (82, 100) and thereby releasing it forcibly when the rotating basic shaft (78) lies near a zero point.

11. A blood pressure measuring apparatus (1) for measuring a blood pressure level of a subject to be measured, the apparatus **characterized by** comprising:
the blood pressure level display device (10) according to any one of claims 1 to 10; and
pressure control means (14, 14A, 14B) for applying and reducing pressure to the subject to be measured,
wherein the blood pressure level display device (10) further includes motion converting means (60, 62, 68, 72) for converting a value of pressure the pressure control means (14, 14A, 14B) applies to the subject to be measured into a rotational motion, and using the rotational motion as a power source for rotating the high blood pressure side indicator needle (26) and the low blood pressure side indicator needle (28).

12. A blood pressure level display method of using a blood pressure display panel (24) provided with a circumferentially-calibrated scale (24A) intended for blood pressure levels, and rotating an indicator needle (26, 28) for displaying a blood pressure level so that the blood pressure level is visually identifiable from the scale (24A) indicated by the indicator needle (26, 28), the method **characterized by** comprising:
a systolic blood pressure indicating step of providing a systolic blood pressure indication based on a state of systolic blood pressure;
a diastolic blood pressure indicating step of providing a diastolic blood pressure indication based on a state of diastolic blood pressure;
a systolic blood pressure display step of rotating a high blood pressure side indicator needle (26) in conjunction with a state of pressure applied to a subject to be measured, and stopping the high blood pressure side indicator needle (26) based on the systolic blood pressure indication from the systolic blood pressure indicating unit (30), thereby displaying at rest a systolic blood pressure level on the scale (24A) pointed by the high blood pressure side indicator needle (26); and
a diastolic blood pressure display step of rotating a low blood pressure side indicator needle (28) in conjunction with the state of pressure applied to the subject to be measured, and stopping the low blood pressure side indicator needle (28) based on the diastolic blood pressure indication from the diastolic blood pressure indicating unit (30), thereby displaying at rest a diastolic blood pressure level on the scale (24A) pointed by the low blood pressure side indicator needle (28).
